# EUROPEAN PATENT APPLICATION

(11) **EP 4 147 777 A1**
(43) Date of publication of application: **15.03.2023**
(21) Application number: 22191992.1
(22) Date of filing: 24.08.2022
(51) Int. Cl.: B01J 35/00, B01J 21/06, B01J 23/40, B01J 35/04, A61L 9/20

(54) **METAL FOAM PHOTOCATALYST APPARATUS, AND VEHICLE HEATING VENTILATION AIR CONDITIONING APPARATUS INCLUDING THE SAME**

(30) Priority: 10.09.2021 KR 20210121140
(71) Applicant: Alantum, Seongnam-si, Gyeonggi-do 13229 (KR)
(72) Inventor: LEE, Jeongmin, Gunpo-si, Gyeonggi-do (KR); HONG, Seung Hyun, Gijang-gun, Busan (KR)
(74) Representative: Manitz Finsterwald Patent- und Rechtsanwaltspartnerschaft mbB

(57) **Abstract**

Disclosed is a metal foam photocatalyst apparatus including: a substrate; a light source disposed on the substrate and irradiating light; a metal foam covering the light source on the substrate and having a curved shape, and a photocatalyst disposed on the metal foam and reacting to the light source, in which the metal foam photocatalyst apparatus removes one or more of bad odors, microorganisms, molds, or viruses contained in the air passing through the metal foam by the reaction of the photocatalyst.

## Description

### CROSS-REFERENCE TO RELATED APPLICATION

This application claims priority to and the benefit of Korean Patent Application No. 10-2021-0121140 filed in the Korean Intellectual Property Office on September 10, 2021, the entire contents of which are incorporated herein by reference.

### BACKGROUND OF THE INVENTION

### (a) Field of the Invention

A metal foam photocatalyst apparatus, and a vehicle Heating Ventilation Air Conditioning (HVAC) apparatus including the same are provided.

### (b) Description of the Related Art

In general, a plasma type ionizer is installed inside a vehicle Heating, Ventilation, Air Conditioning (HVAC) apparatus. The ionizer generates active oxygen to remove bad odors inside the vehicle, but at the same time, the ionizer generates ozone that is a carcinogen. Due to ozone regulation, the output of the plasma type ionizer is limited, and consequently the effect of removing bad odors is also reduced.

In order to remove the bad odor inside the vehicle without generating ozone, a photocatalyst apparatus installed inside the vehicle HVAC apparatus may be used. Such a photocatalyst apparatus utilizes an ultraviolet light source, a TiO₂ catalyst is applied to a planar honeycomb carrier, and a heat dissipation mechanism or a waterproof mechanism is used together. However, in a photocatalyst apparatus using an ultraviolet light source, heat is generated due to ultraviolet rays, and a heat dissipation mechanism is inevitably used together.

The above information disclosed in this Background section is only for enhancement of understanding of the background of the invention, and therefore it may contain information that does not form the prior art that is already known in this country to a person of ordinary skill in the art.

### SUMMARY OF THE INVENTION

An exemplary embodiment has been made in an effort to remove bad odor in a vehicle interior and inside a vehicle Heating, Ventilation, Air Conditioning (HVAC) apparatus, suppress microbial growth, and remove mold and viruses.

In addition to the above problems, the exemplary embodiment may be used to achieve other tasks not specifically mentioned.

An exemplary embodiment provides a metal foam photocatalyst apparatus including: a substrate; a light source disposed on the substrate and irradiating light; a metal foam covering the light source on the substrate and having a curved shape, and a photocatalyst disposed on the metal foam and reacting to the light source, in which the metal foam photocatalyst apparatus removes one or more of bad odors, microorganisms, molds, or viruses contained in the air passing through the metal foam by the reaction of the photocatalyst.

The light may be visible light or natural light.

A noble metal may be applied on the photocatalyst.

Alumina, ceria, zeolite, or one or more thereof may be disposed in the metal foam.

The metal foam may have a dome shape.

The metal foam may have a cylinder shape.

The metal foam may have a semi-cylinder shape.

The metal foam photocatalyst apparatus may further include a metal plate attached to the metal foam photocatalyst apparatus.

An exemplary embodiment provides a vehicle Heating, Ventilation, Air Conditioning (HVAC) apparatus, including: a metal foam photocatalyst apparatus installed inside or outside, in which the metal foam photocatalyst apparatus includes: a substrate; a light source disposed on the substrate and irradiating light; a metal foam covering the light source on the substrate and having a curved shape, and a photocatalyst disposed on the metal foam and reacting to the light source, and the metal foam photocatalyst apparatus removes one or more of bad odors, microorganisms, molds, or viruses contained in the air passing through the metal foam by the reaction of the photocatalyst.

The metal foam photocatalyst apparatus may be attached to an air confluence part or an air outlet part of the vehicle HVAC apparatus.

In the vehicle HVAC apparatus, the metal foam photocatalyst apparatus may radiate heat without a heat radiation substrate or a heat radiation fin.

According to the exemplary embodiments, the metal foam photocatalyst apparatus may be applied to the inside or outside of the vehicle HVAC apparatus, and may be applied to a discharge surface or a flow confluence part.

According to the exemplary embodiments, the metal foam photocatalyst apparatus may utilize natural light, and a heat radiation device or a waterproof device may not be used.

According to the exemplary embodiments, the metal foam photocatalyst apparatus uses metal foam having a high porosity as a catalyst carrier, thereby maximizing the specific surface area and maximizing the performance and maximizing the light transmittance.

According to the exemplary embodiments, the metal foam photocatalyst apparatus uses metal foam with high flexibility as a catalyst carrier, so that it is possible to achieve high processability, make it possible to uniformize the light irradiation distance, maximize the performance by maximizing the specific surface area, maximize the light transmittance, and allow a reflective plate to be attached.

### BRIEF DESCRIPTION OF THE DRAWINGS

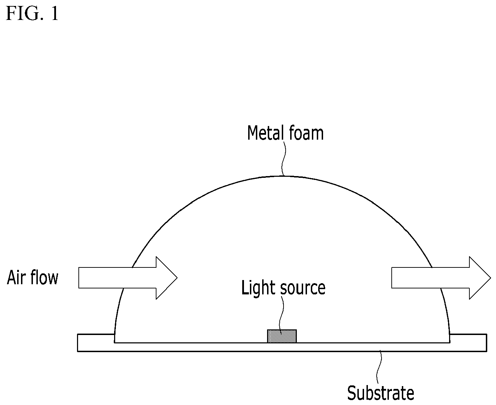

FIG. 1 is a diagram schematically illustrating a metal foam photocatalyst apparatus according to an exemplary embodiment.

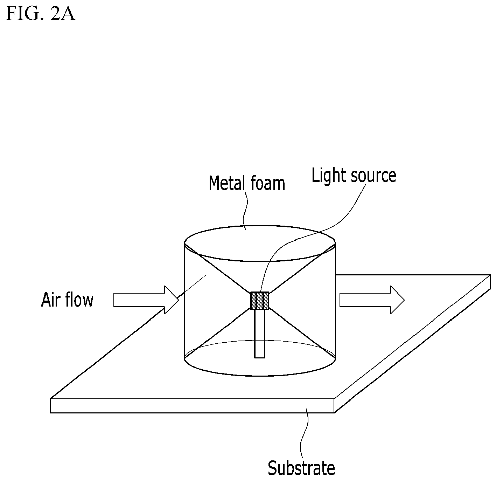

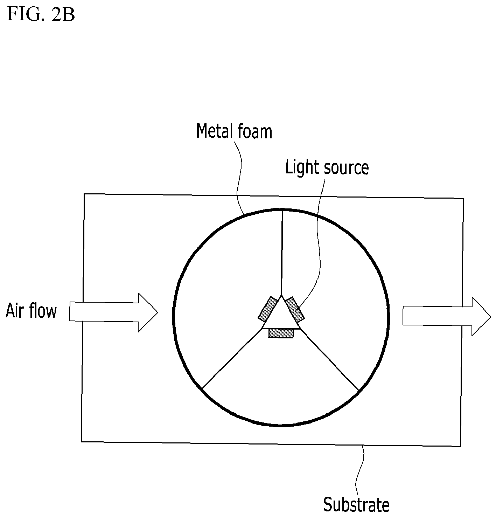

FIG. 2A is a perspective view schematically illustrating the metal foam photocatalyst apparatus according to the exemplary embodiment, and FIG. 2B is a top plan view schematically illustrating the metal foam photocatalyst apparatus according to the exemplary embodiment.

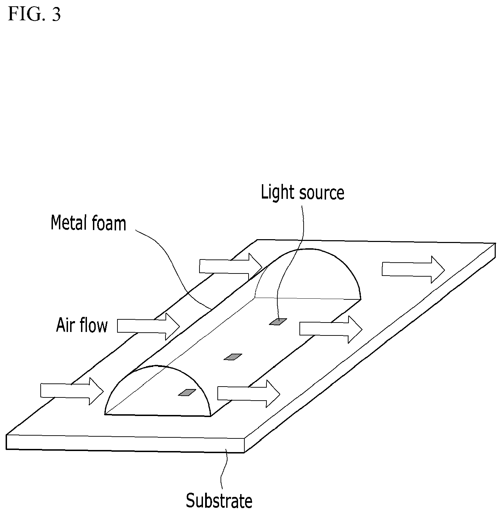

FIG. 3 is a diagram schematically illustrating the metal foam photocatalyst apparatus according to the exemplary embodiment.

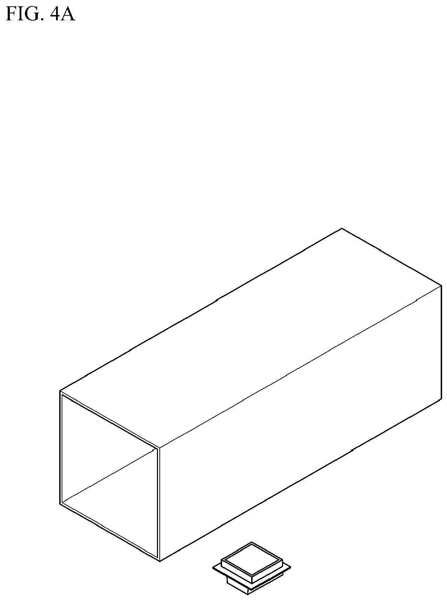

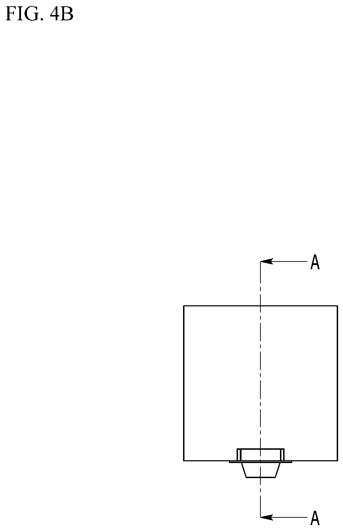

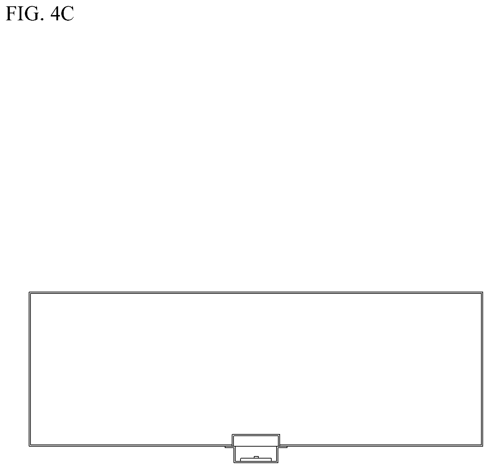

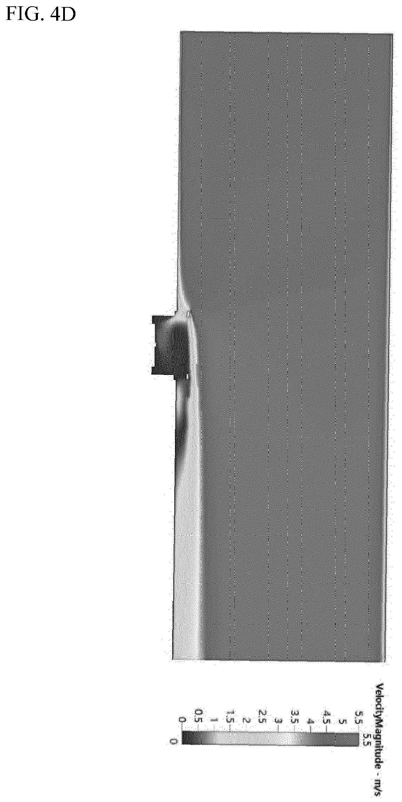

FIG. 4A is a diagram schematically illustrating a photocatalyst apparatus in the related art, FIG. 4B is a side view of the photocatalyst apparatus in the related art, FIG. 4C is a cross-sectional view taken along line A-A of FIG. 4B, and FIG. 4D is a diagram illustrating a flow rate analysis result for the photocatalyst apparatus in the related art of FIGS. 4A to 4C.

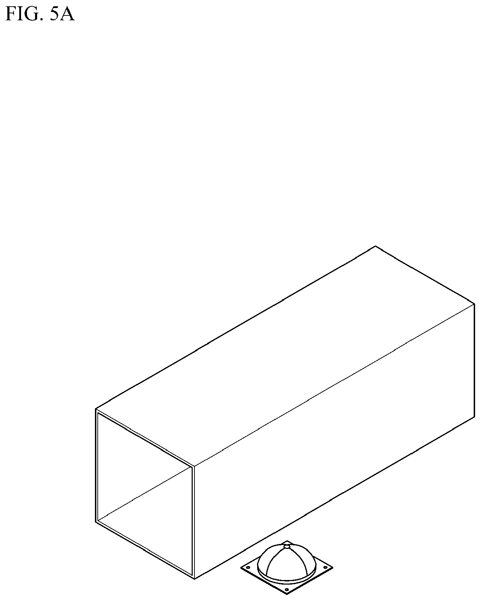

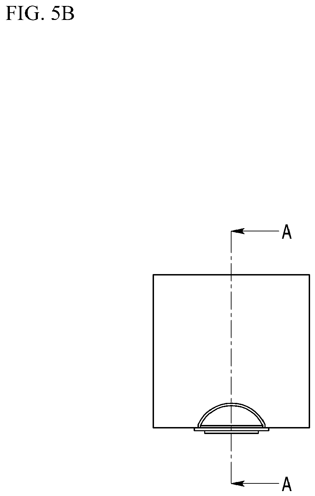

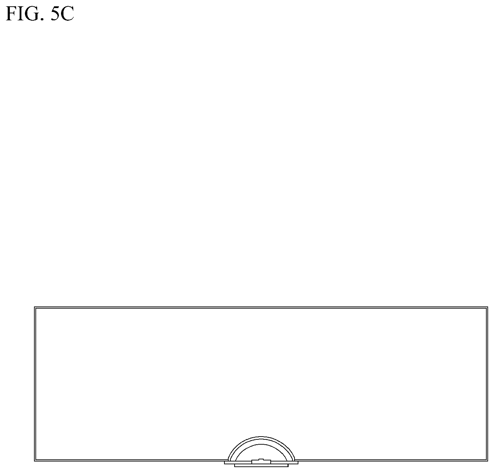

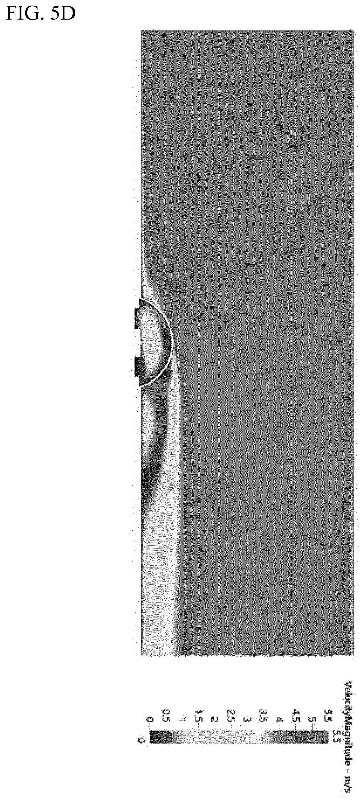

FIG. 5A is a diagram schematically illustrating a photocatalyst apparatus in the related art, FIG. 5B is a side view of the photocatalyst apparatus in the related art, FIG. 5C is a cross-sectional view taken along line A-A of FIG. 5B, and FIG. 5D is a diagram illustrating a flow rate analysis result for the metal form photocatalyst apparatus in the related art of FIGS. 5A to 5C.

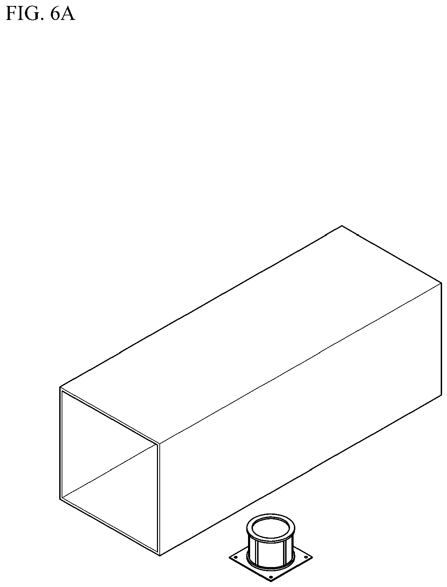

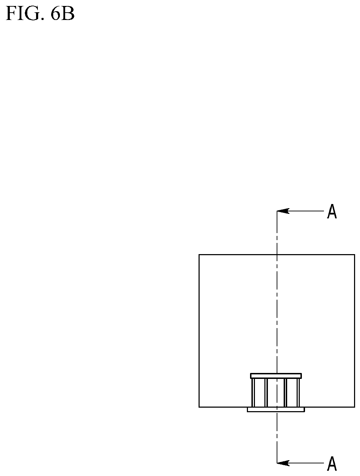

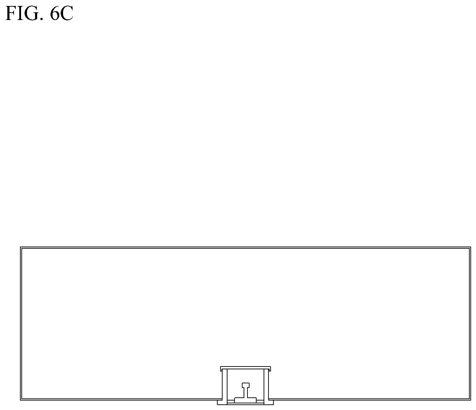

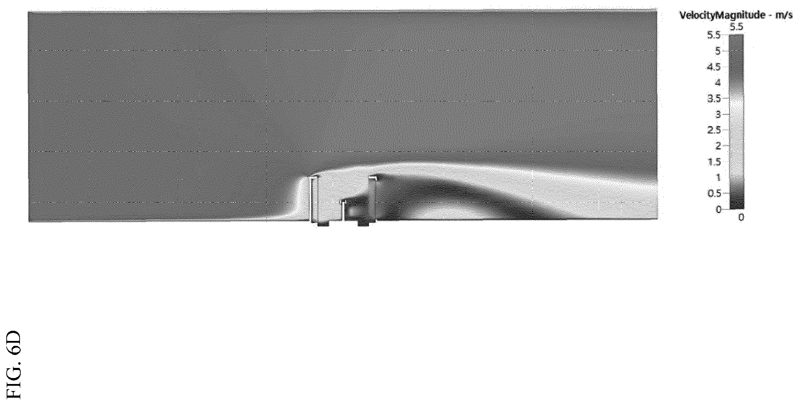

FIG. 6A is a diagram schematically illustrating a photocatalyst apparatus in the related art, FIG. 6B is a side view of the photocatalyst apparatus in the related art, FIG. 6C is a cross-sectional view taken along line A-A of FIG. 6B, and FIG. 6D is a diagram illustrating a flow rate analysis result for the metal foam photocatalyst apparatus in the related art of FIGS. 6A to 6C.

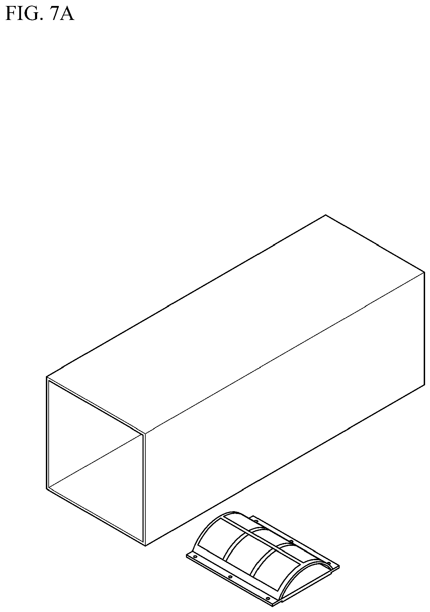

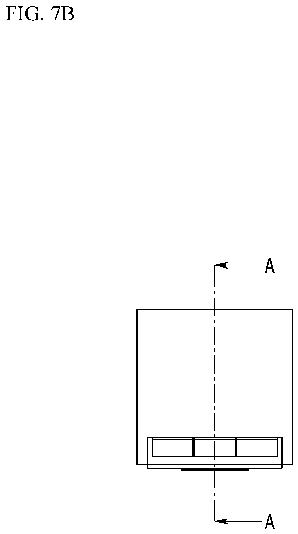

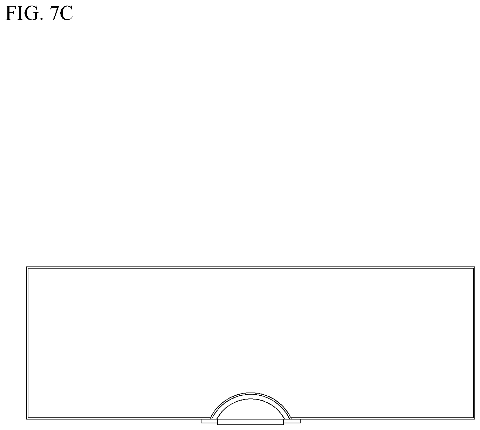

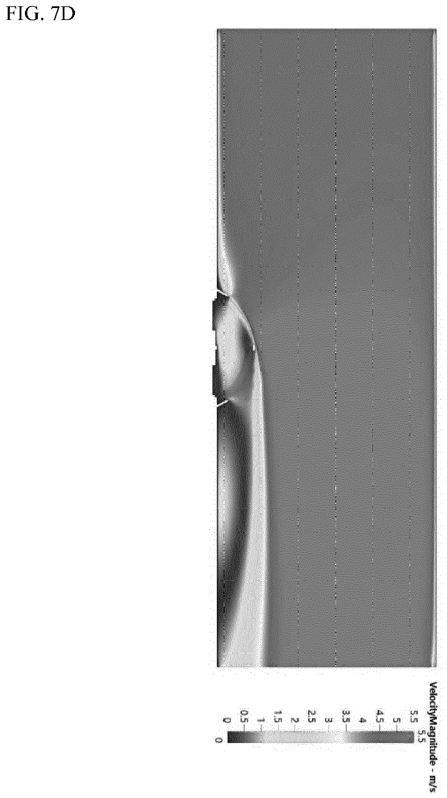

FIG. 7A is a diagram schematically illustrating a photocatalyst apparatus in the related art, FIG. 7B is a side view of the photocatalyst apparatus in the related art, FIG. 7C is a cross-sectional view taken along line A-A of FIG. 7B, and FIG. 7D is a diagram illustrating a flow rate analysis result for the metal foam photocatalyst apparatus in the related art of FIGS. 7A to 7C.

### DETAILED DESCRIPTION OF THE EXEMPLARY EMBODIMENTS

In the following detailed description, only certain exemplary embodiments of the present invention have been illustrated and described, simply by way of illustration. However, the present invention can be variously implemented and is not limited to the following embodiments. Accordingly, the drawings and description are to be regarded as illustrative in nature and not restrictive. Like reference numerals designate like elements throughout the specification. In addition, in the case of a well-known published technology, a detailed description thereof will be omitted.

Throughout the specification, unless explicitly described to the contrary, the word "comprise", and variations such as "comprises" or "comprising", will be understood to imply the inclusion of stated elements but not the exclusion of any other elements.

Then, a metal foam photocatalyst apparatus according to an exemplary embodiment will be described in detail.

FIG. 1 is a diagram schematically illustrating a metal foam photocatalyst apparatus according to an exemplary embodiment, FIG. 2A is a perspective view schematically illustrating the metal foam photocatalyst apparatus according to the exemplary embodiment, and FIG. 2B is a top plan view schematically illustrating the metal foam photocatalyst apparatus according to the exemplary embodiment, and FIG. 3 is a diagram schematically illustrating the metal foam photocatalyst apparatus according to the exemplary embodiment.

Referring to FIGS. 1 to 3, a light source is disposed on a substrate, and a curved metal foam covers the light source. Air penetrates through the metal foam, and a photocatalyst formed in the metal foam reacts through the light source, thereby removing bad odors, microorganisms, mold, viruses, and the like contained in the air.

For example, the metal foam may be used as a catalyst carrier, and the porosity of the metal foam may be 90% or more, and accordingly, the performance of the photocatalyst apparatus may be improved through maximization of the specific surface area. In addition, the specific surface area of the metal foam may be about 1.1 m²/L to about 13 m²/L, and in this case, the light transmittance of the metal foam is maximized, and thus the performance of the photocatalyst apparatus may be maximized. In addition, the metal foam may include one or more metals such as nickel, iron, chromium, and aluminum to be manufactured thinly. Since the metal foam has flexibility, workability is high, and the metal foam may be manufactured in a curved shape to easily achieve uniformity of the light irradiation distance. For example, the curved shape may have a dome shape of FIG. 1, a cylindrical shape of FIGS. 2A and 2B, and a semi-cylindrical shape of FIG. 3. Also, the dome shape may be a spherical shape, a hexagonal shape, or an octagonal shape. Due to the curved shape of the metal foam, the light irradiation distance from the light to the metal foam may be equal at various positions. In addition, the curved shape of the metal foam may make the light penetration depth thin, and thus, the volume of air processed by the photocatalyst apparatus may be maintained, and the pressure drop may be maintained at a low level at the same time. In addition, when the photocatalyst apparatus is used in a vehicle Heating, Ventilation, Air Conditioning (HVAC) apparatus, the photocatalyst apparatus may not be installed on the inclined portion.

For example, the photocatalyst may include TiO₂. A noble metal, such as platinum, rhodium, or palladium, may be applied on the photocatalyst, and in this case, the activation energy is further lowered. In addition, the photocatalyst may be used together with a cocatalyst, such as alumina, ceria, or zeolite.

For example, the light source may include one or more visible light or natural light, and ozone generation due to ultraviolet light may be prevented. An LED may be used as the light source. In addition, since visible light or natural light generates less heat than ultraviolet light, the use of a heat radiating substrate or heat radiating fin is not required. Furthermore, in order to compensate for the low energy of visible light or natural light to remove bad odors, microorganisms, mold, viruses, and the like in the air, a noble metal may be applied on the photocatalyst, or a cocatalyst may be used together with the photocatalyst.

In addition, a reflector may be attached to the photocatalyst apparatus, and in this case, the performance of the photocatalyst apparatus may be further improved.

The metal foam photocatalyst apparatus described above is applicable to the inside or outside of the vehicle HVAC apparatus, and is applicable to the discharge surface or the flow confluence part.

For example, when the curved metal foam is compared with a flat honeycomb in the related art, the curved metal foam has a larger specific surface area than the flat honeycomb in the related art, and thus the volume thereof is reduced. In addition, in the case of the curved metal foam, since the thickness is reduced compared to the same volume, the pressure drop may also be reduced. In addition, in the case of the curved metal foam, due to the consistent flow of air, the pressure drop may be reduced, the amount of inflow and outflow of air may be increased, and noise may be reduced. In addition, in the case of the curved metal foam, since the light irradiation length is uniform, the decomposition performance of the photocatalyst apparatus may be improved.

Flow analysis for a photocatalyst apparatus using the flat honeycomb in the related art is illustrated in FIGS. 4A to 4D. For the flow analysis using the curved metal foam according to the exemplary embodiment, a flow analysis for a metal foam photocatalyst apparatus in a dome shape is illustrated in FIGS. 5A to 5D, a flow analysis for a metal foam photocatalyst apparatus in a cylindrical shape is illustrated in FIGS. 6A to 6D, and a flow analysis for a metal foam photocatalyst apparatus in a semi-cylindrical shape is illustrated in FIGS. 7A to 7D. The flow analysis results of the flow analysis are illustrated in Table 1 below. The injected air has a velocity of 5 Am/sec, a flow rate of 455 kg/hr, and a temperature of 25°C. The volume of air is the same in the flat honeycomb photocatalyst apparatus in the related art, the dome-type metal foam photocatalyst apparatus, and the cylindrical metal foam photocatalyst apparatus, and the volume of air in the semi-cylindrical metal foam photocatalyst apparatus is about 3 times larger than the volume of air in the flat honeycomb photocatalyst apparatus in the related art.

**(Table 1)**

| | Flow analysis result | Catalyst Flow | | Difference (%) | |
|---|---|---|---|---|---|
| | Total ΔP(Pa) | Flow Rate(kg/hr) | Portion (based on Inlet flow rate) | Pressure Drop | Flow Rate |
| Flat shape | 2.09 | 5.11 | 1.12% | 100% | 100% |
| Dome shape | 2.46 | 7.93 | 1.74% | 118% | 155% |
| Cylinder shape | 3.32 | 12.63 | 2.78% | 159% | 247% |
| Semi-cylinder shape | 5.99 | 12.90 | 21.6% | 286% | 505% |

Referring to Table 1, in comparison with the flat photocatalyst apparatus in the related art, the curved metal foam photocatalyst apparatus according to the exemplary embodiment increases the distribution flow rate of air into the catalyst, thereby improving catalytic performance. In addition, as the distribution flow rate of air increases, the rise width of the pressure drop is small. In the case of the semi-cylindrical metal foam photocatalyst apparatus, the increase in the pressure drop is 3 times or less, and the distribution flow rate may be increased by about 5 times, even though the volume is increased 3 times compared to the flat photocatalyst apparatus in the related art. Accordingly, in the curved metal foam photocatalyst apparatus according to the exemplary embodiment, an increase in pressure drop may be minimized while an air distribution flow rate is increased.

Then, a vehicle HVAC apparatus according to an exemplary embodiment will be described in detail.

The vehicle HVAC apparatus includes a metal foam photocatalyst apparatus installed inside or outside. The foregoing descriptions with reference to FIGS. 1 to 7D may be equally applied to the description of the metal foam photocatalyst apparatus.

For example, the metal foam photocatalyst apparatus includes a substrate, a light source disposed on the substrate and irradiating light, a metal foam covering the light source on the substrate and having a curved shape, and a photocatalyst located on the metal foam and reacting to the light source, and removes one or more of bad odors, microorganisms, mold, or viruses contained in the air passing through the metal foam by the reaction of the photocatalyst.

The metal foam photocatalyst apparatus may be attached to the air confluence part or the air outlet part of the vehicle HVAC apparatus.

In the vehicle HVAC apparatus, the metal foam photocatalyst apparatus may radiate heat without a heat radiation substrate or heat radiation fin.

While this invention has been described in connection with what is presently considered to be practical exemplary embodiments, it is to be understood that the invention is not limited to the disclosed embodiments. On the contrary, it is intended to cover various modifications and equivalent arrangements included within the spirit and scope of the appended claims.

## Claims

1. A metal foam photocatalyst apparatus, comprising:
a substrate;
a light source disposed on the substrate and irradiating light;
a metal foam covering the light source on the substrate and having a curved shape, and
a photocatalyst disposed on the metal foam and reacting to the light source
wherein the metal foam photocatalyst apparatus removes one or more of bad odors, microorganisms, molds, or viruses contained in the air passing through the metal foam by the reaction of the photocatalyst.

2. The metal foam photocatalyst apparatus of claim 1, wherein:
the light is visible light or natural light.

3. The metal foam photocatalyst apparatus of claim 2, wherein:
a noble metal is applied on the photocatalyst.

4. The metal foam photocatalyst apparatus of claim 2, wherein:
alumina, ceria, zeolite, or one or more thereof are disposed in the metal foam.

5. The metal foam photocatalyst apparatus of claim 1, wherein:
the metal foam has a dome shape.

6. The metal foam photocatalyst apparatus of claim 1, wherein:
the metal foam has a cylinder shape.

7. The metal foam photocatalyst apparatus of claim 1, wherein:
the metal foam has a semi-cylinder shape.

8. The metal foam photocatalyst apparatus of claim 1, further comprising:
a metal plate attached to the metal foam photocatalyst apparatus.

9. A vehicle Heating, Ventilation, Air Conditioning (HVAC) apparatus, comprising:
a metal foam photocatalyst apparatus installed inside or outside,
wherein the metal foam photocatalyst apparatus includes:
a substrate;
a light source disposed on the substrate and irradiating light;
a metal foam covering the light source on the substrate and having a curved shape, and
a photocatalyst disposed on the metal foam and reacting to the light source, and
the metal foam photocatalyst apparatus removes one or more of bad odors, microorganisms, molds, or viruses contained in the air passing through the metal foam by the reaction of the photocatalyst.

10. The vehicle HVAC apparatus of claim 9, wherein:
the metal foam photocatalyst apparatus is attached to an air confluence part or an air outlet part of the vehicle HVAC apparatus.

11. The vehicle HVAC apparatus of claim 9, wherein:
the metal foam photocatalyst apparatus radiates heat without a heat radiation substrate or without a heat radiation fin.
